# EUROPEAN PATENT SPECIFICATION

(11) **EP 3 302 715 B1**
(45) Date of publication and mention of the grant of the patent: **15.04.2020**
(21) Application number: 16726889.5
(22) Date of filing: 03.06.2016
(51) Int. Cl.: A61Q 5/06, A61K 8/81, A61K 8/86, A61K 8/04

(54) **COMPOSITION COMPRISING A PARTICULAR ANIONIC ASSOCIATIVE POLYMER, A PARTICULAR FIXING POLYMER AND A PARTICULAR ANIONIC POLYMER**
ZUSAMMENSETZUNG MIT EINEM BESTIMMTEN ANIONISCHEN ASSOZIATIVEN POLYMER, EINEM BESTIMMTEN FIXIERPOLYMER UND EINEM BESTIMMTEN ANIONISCHEN POLYMER
COMPOSITION COMPRENANT UN POLYMÈRE ASSOCIATIF ANIONIQUE PARTICULIER, UN POLYMÈRE FIXATEUR PARTICULIER ET UN POLYMÈRE ANIONIQUE PARTICULIER

(30) Priority: 04.06.2015 FR 1555097
(43) Date of publication of application: 11.04.2018
(73) Proprietor: L'Oréal, 75008 Paris (FR)
(72) Inventor: GILLES, Audrey, 92110 Clichy (FR)
(74) Representative: Casalonga
(86) International application number: PCT/EP2016/062707
(87) International publication number: WO 2016/193467

(56) References cited:
- FR-A1- 2 984 135
- US-A1- 2014 093 467
- Anonymous: "Ashland brings performance and style to crystal clear gel with AquaStyle", , 1 April 2014 (2014-04-01), XP002751471, Retrieved from the Internet: URL:http://files.shareholder.com/downloads /ASH/0x0x739330/f61f8ac2-932b-4e99-afa7-0d 1962337028/ASH_News_2014_4_1_Products_and_ Services.pdf [retrieved on 2015-11-26]
- "Personal Care and Cosmetic Compositions Comprising Acrylates Copolymers", IP.COM JOURNAL, IP.COM INC., WEST HENRIETTA, NY, US, 20 May 2015 (2015-05-20), XP013167428, ISSN: 1533-0001

## Description

The present invention relates to a composition comprising at least one particular anionic associative polymer, one particular fixing polymer and one particular anionic polymer different from the anionic associative polymer and from the anionic fixing polymer.

The invention also relates to a method for treating keratin fibres, in particular human keratin fibres such as the hair, using the composition according to the invention.

The invention also relates to the use of said composition for styling keratin fibres, preferably the hair.

Styling products are usually used to construct and structure the hairstyle and to give it long-lasting hold. These compositions generally comprise one or more fixing film-forming polymers, in a cosmetically acceptable medium. These polymers allow the formation of a coating film on the hair in order to provide form retention of the hairstyle.

Styling products are generally in the form of lacquers, mousses or else gels. US 2014/0093467 A1 and FR 2984135 A1 disclose hair gels comprising mixtures of anionic polymers.

In particular, styling gels are often used in order to obtain strong fixing of the hairstyle.

These styling gels are solutions of one or more fixing film-forming polymers, thickened or gelled with one or more thickening polymers.

These thickening polymers are introduced in an attempt to give the composition good usage qualities such as easy uptake in the hands, better distribution and good spreading on the hair.

However, the strong fixing styling gels of the prior art generally have several drawbacks. They are especially difficult to distribute both on the hands and/or the hair and do not spread out sufficiently over the whole of the head of hair. In addition, the fixing obtained is sometimes insufficient and the hold of the hairstyle over time is not completely satisfactory, the film formed by the gel having a tendency to disintegrate over time.

There is therefore a real need to formulate a composition, especially a styling composition that enables a high level of fixing, with an improved durability of the hair shaping and which is easy to distribute on the hands and/or the hair.

It has now been discovered that the combination of one or more particular anionic associative polymers, one or more particular fixing polymers, and one or more particular anionic polymers different from those mentioned makes it possible to obtain a cosmetic composition which has high styling performance, especially in terms of level of fixing and of durability of the hair shaping, while retaining good usage qualities. In particular, the compositions according to the invention have an improved resistance, especially an improved resistance to mechanical friction.

One subject of the present invention is thus especially a composition comprising:
- one or more particular anionic associative polymers (i) as defined below,
- one or more particular anionic fixing polymers (ii) as defined below,
- one or more anionic polymers (iii), different from the anionic associative polymers (i) and from the anionic fixing polymers (ii) as defined below.

The composition according to the invention thus has high styling performance, and in particular great fixing power and long-lasting hold of the hairstyle treated with this composition with an improved feel. A flexible hairstyle is also obtained, and also a long-lasting flexibility. Furthermore, shiny hair is obtained.The gel obtained has also an improved resistance to friction which is not rapidly eroded.

The composition according to the invention also has good usage qualities. In particular it has a texture which enables it to facilitate the grasping thereof and the distribution thereof over the head of hair.

Another subject of the present invention is the use of the abovementioned composition for styling keratin fibres, preferably the hair.

Yet another subject of the invention is a method for treating keratin fibres, in particular human keratin fibres such as the hair, comprising the application of the abovementioned composition to said keratin fibres.

Other subjects, characteristics, aspects and advantages of the invention will become even more clearly apparent on reading the description and the examples which follow.

In the text hereinbelow, and unless otherwise indicated, the limits of a range of values are included in that range, in particular in the expressions "between" and "ranging from ... to ...".

In addition, the expression "at least one" is equivalent to the expression "one or more".

For the purposes of the present invention, the term "associative polymer" means an amphiphilic polymer that is capable, in an aqueous medium, of reversibly combining with itself or with other molecules. It generally comprises, in its chemical structure, at least one hydrophilic region or group and at least one hydrophobic region or group.

For the purposes of the present invention, the term "hydrophobic group" means a group or a polymer bearing a saturated or unsaturated, linear or branched hydrocarbon-based chain. When it denotes a hydrocarbon-based group, the hydrophobic group comprises at least 8 carbon atoms, preferably from 8 to 50 carbon atoms, in particular from 10 to 30 carbon atoms and preferentially from 12 to 22 carbon atoms. Preferentially, the hydrocarbon-based hydrophobic group originates from a monofunctional compound. By way of example, the hydrophobic group may be derived from a fatty alcohol, such as stearyl alcohol, dodecyl alcohol or decyl alcohol, or else from a polyalkylenated fatty alcohol, such as Steareth-100. It may also denote a hydrocarbon-based polymer, for instance polybutadiene.

In other words, the anionic associative polymer is an anionic amphiphilic polymer.

For the purposes of the present invention, the term "fixing polymer" means a polymer that is capable of giving a head of hair a shape and/or of holding the head of hair in a given shape.

As explained previously, the composition according to the invention comprises at least one particular anionic associative polymer (i).

The anionic associative polymer (i) comprises at least one unit of formula (I) and at least one unit of formula (II) below: 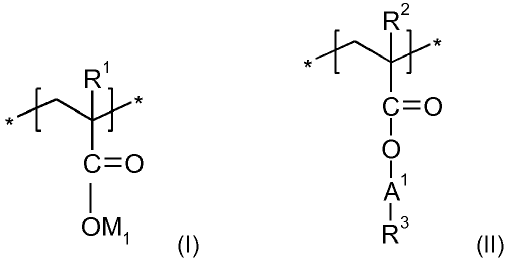
with R¹ and R², independently of each other, representing a hydrogen atom or a methyl group,
R³ representing a C₈-C₃₀ alkyl radical,
M₁ representing a hydrogen atom or a physiologically acceptable cation, and
A¹ representing a group -(CH₂CH₂O)ₓ-, x being an integer ranging from 5 to 35, a group -(CH₂CHMeO)_{y}-, y being an integer ranging from 5 to 35, or a group - (CH₂CH₂O)ₓ-(CH₂CHMeO)_{y}-, the sum x + y being an integer ranging from 5 to 35 with x and y being greater than 0.

As C₈ to C₃₀ alkyl radical according to the invention, mention may be made especially of the following radicals: octyl (capryl), decyl (caprinyl), dodecyl (lauryl), tetradecyl (myristyl), hexadecyl (cetyl), octadecyl (stearyl), eicosyl (arachidyl) and docosyl (behenyl).

In the above formulae and in all the following formulae, a chemical bond indicated by the symbol * corresponds to a free valency of the structural fragment in question.

As physiologically acceptable cation in formula (I) above and also in the other formulae of the description, mention may be made especially of cations of the physiologically acceptable metals of groups la, Ib, Ila, IIb, IIIb, VIa and VIII of the Periodic Table of the Elements, ions of ammonium type and cationic organic compounds comprising a quaternized nitrogen atom. The latter are formed, for example, by protonation, using an acid, of a primary, secondary or tertiary organic amine, or else by permanent quaternization of such organic amines. Examples of these cationic organic ammonium compounds are 2-ammonioethanol and 2-trimethylammonioethanol.

Preferably, M₁ denotes a hydrogen atom, a sodium ion or an ion derived from an alkanolamine.

In a first embodiment of the invention, the anionic associative polymer(s) (i) that can be used in the composition according to the invention is (are) chosen from those in which, in formula (II), A¹ represents a group of formula -(CH₂CH₂O)ₓ-, x being an integer ranging from 5 to 35 and in particular ranging from 10 to 24.

In a second embodiment of the invention, the anionic associative polymer(s) (i) comprise(s) a unit of formula (II) above in which R³ represents a C₁₂-C₂₀ alkyl radical.

In a third embodiment of the invention, the anionic associative polymer(s) (i) that can be used in the composition according to the invention is (are) chosen from those in which, in formula (II), R² represents a methyl group.

In a fourth embodiment of the invention, the anionic associative polymer(s) (i) comprise(s) at least one unit of formula (I) as defined above and at least one unit of formula (II') below: 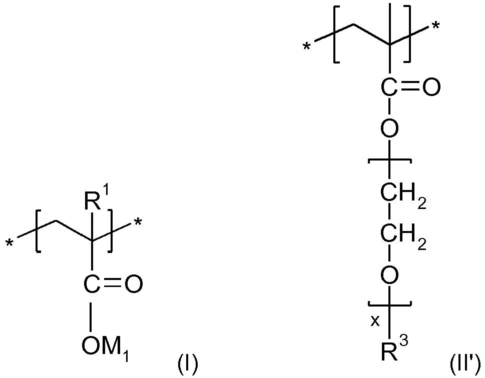 with R¹ representing a hydrogen atom or a methyl group, M₁ representing a hydrogen atom or a physiologically acceptable cation, R³ representing a C₈ to C₃₀ alkyl radical and x being an integer ranging from 5 to 35 and preferably ranging from 10 to 24.

Preferably, in the composition according to the invention, the anionic associative polymer(s) (i) are crosslinked.

For the purposes of the invention, the terms "crosslinked" and "crosslinking" should be understood as meaning the interconnection of the polymer chains via covalent chemical bonds, with formation of a network. This covalent connection of the polymer chains may be performed via direct covalent bonds or by means of molecular fragments forming bridges between the polymer chains. Such a molecular fragment is linked via a covalent chemical bond to each of the polymer chains between which it forms a bridge. For the purposes of the invention, the term "non-crosslinked" should be understood as meaning the absence of any "crosslinking" as defined above.

Crosslinking of the anionic associative polymer(s) is generally performed using at least one crosslinking agent.

The crosslinking agent(s) may be chosen especially from polyunsaturated aromatic compounds such as divinylbenzenes, divinylnaphthalenes and trivinylbenzenes, polyunsaturated alicyclic compounds such as 1,2,4-trivinylcyclohexane, phthalic acid diesters such as diallyl phthalate, polyunsaturated aliphatic compounds such as dienes, trienes and tetraenes, for instance isoprene, 1,3-butadiene, 1,5-hexadiene, 1,5,9-decatriene, 1,9-decadiene and 1,5-heptadiene, polyalkenyl ethers such as triallyl pentaerythritol, diallyl pentaerythritol, diallyl sucrose, octaallyl sucrose and trimethylolpropane diallyl ether, polyunsaturated esters of polyols or of polyacids such as 1,6-hexanediol di(meth)acrylate, tetramethylolmethane tri(meth)acrylate, allyl acrylate, diallyl itaconate, diallyl fumarate, diallyl maleate, trimethylolpropane tri(meth)acrylate, trimethylolpropane di(meth)acrylate and polyethylene glycol di(meth)acrylate such as ethylene glycol di(meth)acrylate, diethylene glycol di(meth)acrylate and triethylene glycol di(meth)acrylate, alkylenebisacrylamides such as methylenebisacrylamide and propylenebisacrylamide, hydroxyl or carboxyl derivatives of methylenebisacrylamide such as N,N'-bis(methylol)methylenebisacrylamide, polyunsaturated silanes such as dimethyldivinylsilane, methyltrivinylsilane, allyldimethylvinylsilane, diallyldimethylsilane and tetravinylsilane, N-methylolacrylamide, N-alkoxy(meth)acrylamides in which the alkoxy group is a C₁-C₁₈ alkoxy group, hydrolysable unsaturated silanes such as triethoxyvinylsilane, triisopropoxyvinylsilane and 3-(triethoxysilyl)propyl methacrylate, hydrolysable silanes such as ethyltriethoxysilane and ethyltrimethoxysilane, hydrolysable silanes bearing an epoxy substituent such as [2-(3,4-epoxycyclohexyl)ethyl]triethoxysilane and (3-glycidyloxypropyl)trimethoxysilane, polyisocyanates such as butane 1,4-diisocyanate, hexane 1,6-diisocyanate, phenylene 1,4-diisocyanate and 4,4'-oxybis(phenyl isocyanate), unsaturated epoxides such as glycidyl methacrylate and allyl glycidyl ether, polyepoxides such as 1,2,5,6-diepoxyhexane diglycidyl ether and ethylene glycol diglycidyl ether, ethoxylated polyols such as diols, triols or diphenols, in each case ethoxylated with 2 to 100 mol of ethylene oxide per mole of hydroxyl groups and bearing, as end group, a polymerizable unsaturated group, for instance a group of vinyl ether, allyl ether, acrylate ester or methacrylate ester type, and acrylate and methacrylate esters of polyols comprising at least two functional groups of acrylate or methacrylate ester type such as trimethylolpropane triacrylate (TMPTA), 15-ethoxylated trimethylolpropane triacrylate (TMPEO15TA), trimethylolpropane dimethacrylate, triethylene glycol dimethacrylate (TEGDMA) and bisphenol A dimethacrylate ethoxylated with 30 mole of ethylene oxide (EOBDMA). Ethoxylated polyols that may especially be mentioned include ethoxylated bisphenol A di(meth)acrylate, ethoxylated bisphenol F di(meth)acrylate and ethoxylated trimethylolpropane tri(meth)acrylate.

In a preferred variant of the invention, the anionic associative polymer(s) (i) comprise(s), in addition to at least one unit of formula (I) and at least one unit of formula (II) or (II'), at least one unit of formula (III) below: 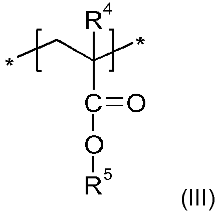
with R⁴ representing a hydrogen atom or a methyl group, and
R⁵ representing a C₁ to C₄ alkyl group, and in particular a methyl or ethyl group. Preferably, R⁵ represents an ethyl group.

In a particularly preferred embodiment of this variant, the anionic associative polymer(s) (i) preferably comprise(s) at least one unit of formula (I) as defined above, at least one unit of formula (II) or (II') as defined above and at least one unit of formula (III) as defined above, with R¹ and R⁴, independently of each other, representing a hydrogen atom or a methyl group, R³ representing a C₈ to C₃₀ alkyl group, R⁵ representing a C₁ to C₄ alkyl group, preferably an ethyl or methyl group, M₁ representing a hydrogen atom or a physiologically acceptable cation, and x being an integer ranging from 5 to 35 and preferably ranging from 10 to 24.

Preferably, M₁ denotes a hydrogen atom, a sodium ion or an ion derived from an alkanolamine.

Preferably, an anionic associative polymer (i) that is most particularly suitable for the composition according to the invention is the acrylate/steareth-20 methacrylate crosslinked copolymer (INCI name: acrylate/steareth-20 methacrylate crosspolymer) which comprises 20 ethylene oxide units. Such a polymer is sold, for example, by the company Rohm & Haas, in the form of a dispersion at 28-30% by weight in water, under the brand name Aculyn® 88.

The particular anionic associative polymer(s) (i) may represent from 0.1% to 20% by weight, preferably from 0.5% to 15% by weight, and in particular from 1% to 10% by weight, better still from 1% to 5% by weight, relative to the total weight of the composition according to the invention.

As explained previously, the composition according to the invention also comprises one or more particular anionic fixing polymers (ii). These anionic fixing polymer(s) (ii) are different from the anionic associative polymers (i) described above.

The anionic fixing polymer(s) (ii) that can be used in the composition according to the invention comprise at least one unit of formula (IV) and at least one unit of formula (V) below: 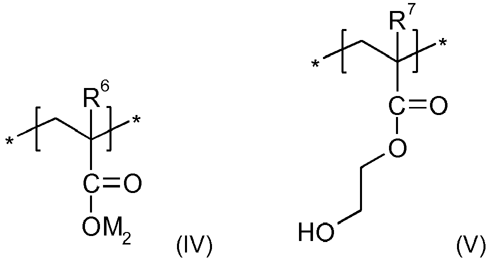
with R⁶ and R⁷, independently of each other, representing a hydrogen atom or a methyl group, and
M₂ representing a hydrogen atom or a physiologically acceptable cation.

As physiologically acceptable cation in formula (IV) above and also in the other formulae of the description, mention may be made especially of cations of the physiologically acceptable metals of groups Ia, Ib, IIa, IIb, IIIb, Via and VIII of the Periodic Table of the Elements, ions of ammonium type and cationic organic compounds comprising a quaternized nitrogen atom. The latter are formed, for example, by protonation, using an acid, of a primary, secondary or tertiary organic amine, or else by permanent quaternization of such organic amines. Examples of these cationic organic ammonium compounds are 2-ammonioethanol and 2-trimethylammonioethanol.

Preferably, M₂ denotes a hydrogen atom, a sodium ion or an ion derived from an alkanolamine.

In a preferred variant of the invention, the anionic fixing polymer(s) (ii) also comprise at least one unit of formula (IV), at least one unit of formula (V), and at least one unit of formula (III) as defined above, with R⁴ representing a hydrogen atom or a methyl group, and R⁵ representing a C₁ to C₄ alkyl group, in particular a methyl, ethyl or n-butyl group.

Preferably, in the composition according to the invention, the anionic fixing polymer(s) (ii) are non-crosslinked.

Preferably, the anionic fixing polymer (ii) that can be used in the composition according to the invention is a copolymer of methacrylic acid/hydroxyethyl methacrylate and of at least one C₁ to C₄ (meth)acrylic acid ester.

In a most particularly preferred manner, the anionic fixing polymer (ii) that can be used in the composition according to the invention is a methacrylic acid/hydroxyethyl methacrylate/methyl methacrylate/butyl acrylate linear tetrapolymer.

Such a polymer is known especially under the trade name Acudyne® 180 and is sold by the company Rohm & Haas.

The anionic fixing polymer(s) (ii) may represent from 0.1% to 20% by weight, preferably from 0.2% to 15% by weight, and in particular from 0.5% to 10%, better still from 1% to 8% by weight, relative to the total weight of the composition according to the invention.

The composition according to the invention also comprises at least one anionic polymer (iii) different from the associative polymers (i) and from the anionic fixing polymers (ii) defined above, which comprises:
- at least one (meth)acrylic acid monomer,
- at least one C₁-C₄ alkyl (meth)acrylate monomer,
- at least one (meth)acrylic ester monomer with a hydrophobic chain.

The anionic polymer (iii) preferably has a molecular weight of between 50 000 and 1 000 000, preferably between 100 000 and 500 000.

The anionic polymer (iii) is preferably crosslinked.

The crosslinking agent(s) may be chosen especially from polyunsaturated aromatic compounds such as divinylbenzenes, divinylnaphthalenes and trivinylbenzenes, polyunsaturated alicyclic compounds such as 1,2,4-trivinylcyclohexane, phthalic acid diesters such as diallyl phthalate, polyunsaturated aliphatic compounds such as dienes, trienes and tetraenes, for instance isoprene, 1,3-butadiene, 1,5-hexadiene, 1,5,9-decatriene, 1,9-decadiene and 1,5-heptadiene, polyalkenyl ethers such as triallyl pentaerythritol, diallyl pentaerythritol, diallyl sucrose, octaallyl sucrose and trimethylolpropane diallyl ether, polyunsaturated esters of polyols or of polyacids such as 1,6-hexanediol di(meth)acrylate, tetramethylolmethane tri(meth)acrylate, allyl acrylate, diallyl itaconate, diallyl fumarate, diallyl maleate, trimethylolpropane tri(meth)acrylate, trimethylolpropane di(meth)acrylate and polyethylene glycol di(meth)acrylate such as ethylene glycol di(meth)acrylate, diethylene glycol di(meth)acrylate and triethylene glycol di(meth)acrylate, alkylenebisacrylamides such as methylenebisacrylamide and propylenebisacrylamide, hydroxyl or carboxyl derivatives of methylenebisacrylamide such as N,N'-bis(methylol)methylenebisacrylamide, polyunsaturated silanes such as dimethyldivinylsilane, methyltrivinylsilane, allyldimethylvinylsilane, diallyldimethylsilane and tetravinylsilane, N-methylolacrylamide, N-alkoxy(meth)acrylamides in which the alkoxy group is a C₁-C₁₈ alkoxy group, hydrolysable unsaturated silanes such as triethoxyvinylsilane, triisopropoxyvinylsilane and 3-(triethoxysilyl)propyl methacrylate, hydrolysable silanes such as ethyltriethoxysilane and ethyltrimethoxysilane, hydrolysable silanes bearing an epoxy substituent such as [2-(3,4-epoxycyclohexyl)ethyl]triethoxysilane and (3-glycidyloxypropyl)trimethoxysilane, polyisocyanates such as butane 1,4-diisocyanate, hexane 1,6-diisocyanate, phenylene 1,4-diisocyanate and 4,4'-oxybis(phenyl isocyanate), unsaturated epoxides such as glycidyl methacrylate and allyl glycidyl ether, polyepoxides such as 1,2,5,6-diepoxyhexane diglycidyl ether and ethylene glycol diglycidyl ether, ethoxylated polyols such as diols, triols or diphenols, in each case ethoxylated with 2 to 100 mol of ethylene oxide per mole of hydroxyl groups and bearing, as end group, a polymerizable unsaturated group, for instance a group of vinyl ether, allyl ether, acrylate ester or methacrylate ester type, and acrylate and methacrylate esters of polyols comprising at least two functional groups of acrylate or methacrylate ester type such as trimethylolpropane triacrylate (TMPTA), 15-ethoxylated trimethylolpropane triacrylate (TMPEO15TA), trimethylolpropane dimethacrylate, triethylene glycol dimethacrylate (TEGDMA) and bisphenol A dimethacrylate ethoxylated with 30 mole of ethylene oxide (EOBDMA). Ethoxylated polyols that may especially be mentioned include ethoxylated bisphenol A di(meth)acrylate, ethoxylated bisphenol F di(meth)acrylate and ethoxylated trimethylolpropane tri(meth)acrylate.

When it is crosslinked, the anionic polymer (iii) may comprise between 0.01% and 4% by weight of crosslinking agent relative to the total weight of the polymer.

Preferably, the anionic polymer (iii) comprises less than 10% by weight of (meth)acrylic ester monomer with a hydrophobic chain relative to the total weight of the polymer.

More preferably, the anionic polymer (iii) comprises between 1% and 5% by weight of (meth)acrylic ester monomer with a hydrophobic chain relative to the total weight of the polymer.

The anionic polymer (iii) may also comprise from 45% to 65% by weight of C₁-C₄ alkyl (meth)acrylate monomer.

It may also comprise from 30% to 50% by weight of (meth)acrylic acid monomer. According to one particular embodiment, the anionic polymer (iii) comprises:
- from 45% to 65% by weight of C₁-C₄ alkyl (meth)acrylate monomer;
- from 30% to 50% by weight of (meth)acrylic acid monomer;
- from 1% to 5% by weight of (meth)acrylic ester monomer with a hydrophobic chain;
- preferably from 0.01% to 4% by weight of crosslinking agent relative to the total weight of the polymer.

Preferably, the C₁-C₄ alkyl (meth)acrylate monomer is an ethyl acrylate monomer.

The (meth)acrylic ester monomer with a hydrophobic chain preferably comprises an oxyalkylenated, saturated or unsaturated, hydrophobic chain.

Preferably, the (meth)acrylic ester monomer with a hydrophobic chain is a (meth)acrylic ester monomer of formula (VI): 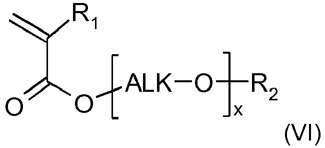 in which
R₁ denotes a hydrogen atom or a linear or branched C₁-C₆ alkyl radical, preferably methyl;
R₂ denotes a saturated or unsaturated, linear or branched, hydrophobic hydrocarbon-based radical comprising from 8 to 50 carbon atoms and more preferentially from 10 to 30 carbon atoms and more preferentially still from 12 to 22 carbon atoms;
ALK is a saturated or unsaturated, linear or branched hydrocarbon-based divalent group comprising from 1 to 10 carbon atoms, preferably from 1 to 6 carbon atoms, preferably 2 or 3 carbon atoms such as the ethylene group,
x denotes an integer ranging from 0 to 100, preferably from 1 to 50, better still from 1 to 20.

According to a particular embodiment of the invention, in formula (VI), the group R₁ represents a methyl.

According to a particular embodiment of the invention, R₂ denotes a saturated or unsaturated, linear or branched, hydrophobic hydrocarbon-based radical preferably comprising from 12 to 22 carbon atoms.

According to one particular embodiment, the anionic polymer (iii) comprises:
- from 45% to 65% by weight of ethyl acrylate monomer;
- from 30% to 50% by weight of (meth)acrylic acid monomer;
- from 1% to 5% by weight of (meth)acrylic ester monomer of formula (VI) in which R₁ represents a methyl, R₂ denotes a saturated or unsaturated, linear or branched, hydrophobic hydrocarbon-based radical preferably comprising from 12 to 22 carbon atoms and ALK represents ethylene;
- preferably from 0.01% to 4% by weight of crosslinking agent relative to the total weight of the polymer.

Preferably, an anionic polymer (iii) that is most particularly suitable for the composition according to the invention is a (meth)acrylic acid/ethyl acrylate/(meth)acrylate with a hydrophobic chain copolymer (INCI name: acrylates copolymer). Such a polymer is for example sold by Ashland under the trade name Aquastyle SH-100.

The anionic polymer(s) (iii) preferably represent(s) from 0.1% to 20% by weight, preferably from 0.5% to 15% by weight, more preferentially from 1% to 10%, and in particular from 1% to 5% by weight, relative to the total weight of the composition.

Preferably, in the composition according to the invention, the weight ratio of the anionic polymer (iii) to the anionic associative polymer (i) ranges from 0.01 to 10, and preferably from 0.05 to 5 and in particular from 0.1 to 3.

Preferably, in the composition according to the invention, the weight ratio of the anionic fixing polymer (ii) to the anionic associative polymer (i) ranges from 0.01 to 10, and preferably from 0.05 to 6 and in particular from 0.1 to 4.

Preferably, in the composition according to the invention, the weight ratio of the anionic polymer (iii) to the anionic fixing polymer (ii) ranges from 0.01 to 10, and preferably from 0.05 to 5 and in particular from 0.1 to 3.

According to one embodiment, the composition according to the present invention comprises:
- one or more anionic associative polymers (i) containing at least one unit of formula (I) as defined above, at least one unit of formula (II') as defined above and at least one unit of formula (III) as defined above,
- one or more anionic fixing polymers (ii) containing at least one unit of formula (IV) as defined above, at least one unit of formula (V) as defined above and at least one unit of formula (III) as defined above,
- one or more anionic polymers (iii), different from the polymers (i) and (ii), comprising at least one ethyl acrylate monomer, at least one (meth)acrylic acid monomer and at least one (meth)acrylic ester monomer of formula (VI) in which R₁ represents a methyl, R₂ denotes a saturated or unsaturated, linear or branched, hydrophobic hydrocarbon-based radical preferably comprising from 12 to 22 carbon atoms.

According to a preferred embodiment, the composition according to the present invention may also comprise one or more surfactants chosen from anionic surfactants, amphoteric or zwitterionic surfactants, nonionic surfactants and cationic surfactants.

The term "anionic surfactant" is intended to mean a surfactant comprising, as ionic or ionizable groups, only anionic groups. These anionic groups are preferably chosen from the following groups: CO₂H, CO₂⁻, SO₃H, SO₃⁻, OSO₃H, OSO₃⁻, H₂PO₃, HPO₃⁻, PO₃²⁻, H₂PO₂, HPO₂⁻, PO₂²⁻, POH, PO⁻.

As examples of anionic surfactants that can be used in the composition according to the invention, mention may be made of alkyl sulfates, alkyl ether sulfates, alkylamido ether sulfates, alkylaryl polyether sulfates, monoglyceride sulfates, alkyl sulfonates, alkylamide sulfonates, alkylaryl sulfonates, α-olefin sulfonates, paraffin sulfonates, alkyl sulfosuccinates, alkyl ether sulfosuccinates, alkylamide sulfosuccinates, alkyl sulfoacetates, acyl sarcosinates, acyl glutamates, alkyl sulfosuccinamates, acyl isethionates and N-(C₁-C₄)alkyl N-acyl taurates, salts of alkyl monoesters of polyglycoside-polycarboxylic acids, acyl lactylates, salts of D-galactoside uronic acids, salts of alkyl ether carboxylic acids, salts of alkylaryl ether carboxylic acids, salts of alkylamido ether carboxylic acids; and the corresponding non-salified forms of all these compounds; the alkyl and acyl groups of all these compounds (unless otherwise mentioned) generally comprising from 6 to 24 carbon atoms and the aryl group generally denoting a phenyl group.

These compounds can be oxyethylenated and then preferably comprise from 1 to 50 ethylene oxide units.

The salts of C₆-C₂₄ alkyl monoesters of polyglycoside-polycarboxylic acids may be chosen from C₆-C₂₄ alkyl polyglycoside-citrates, C₆-C₂₄ alkyl polyglycoside-tartrates and C₆-C₂₄ alkyl polyglycoside-sulfosuccinates.

When the anionic surfactant(s) are in salt form, they may be chosen from alkali metal salts such as the sodium or potassium salt and preferably the sodium salt, ammonium salts, amine salts and in particular amino alcohol salts or alkaline-earth metal salts such as the magnesium salt.

Examples of amino alcohol salts that may especially be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.

Alkali metal or alkaline-earth metal salts and in particular sodium or magnesium salts are preferably used.

The anionic surfactants optionally present may be mild anionic surfactants, i.e. anionic surfactants without a sulfate function.

Mention may in particular be made, as regards the mild anionic surfactants, of the following compounds and their salts, and also mixtures thereof:
polyoxyalkylenated alkyl ether carboxylic acids;
polyoxyalkylenated alkylaryl ether carboxylic acids;
polyoxyalkylenated alkylamido ether carboxylic acids, in particular those comprising from 2 to 50 ethylene oxide groups,
alkyl-D-galactoside uronic acids;
acyl sarcosinates, acyl glutamates; and
alkyl polyglycoside carboxylic esters.

Use may very particularly be made of polyoxyalkylenated alkyl ether carboxylic acids, such as, for example, lauryl ether carboxylic acid (4.5 EO), for example sold under the name Akypo RLM 45 CA from Kao.

The amphoteric or zwitterionic surfactant(s) that can be used in the present invention may especially be optionally quaternized secondary or tertiary aliphatic amine derivatives, in which the aliphatic group is a linear or branched chain containing from 8 to 22 carbon atoms, said amine derivatives containing at least one anionic group, for instance a carboxylate, sulfonate, sulfate, phosphate or phosphonate group.

Mention may be made in particular of (C₈-C₂₀)alkylbetaines, sulfobetaines, (C₈-C₂₀)alkylamido(C₃-C₈)alkylbetaines or (C₈-C₂₀)alkylamido(C₆-C₈)-alkylsulfobetaines.

Among the optionally quaternized secondary or tertiary aliphatic amine derivatives that can be used, as defined above, mention may also be made of the compounds of respective structures (A₁), (A₂) and (A3) below:

Rₐ-CONHCH₂CH₂-N⁺(R_{b})(R_{c})(CH₂COO⁻) (A₁)

in which:
Rₐ represents a C₁₀-C₃₀ alkyl or alkenyl group derived from an acid Rₐ-COOH preferably present in hydrolyzed coconut oil, or a heptyl, nonyl or undecyl group,
R_{b} represents a β-hydroxyethyl group, and
R_{c} represents a carboxymethyl group;
and

Rₐ'-CONHCH₂CH₂-N(B)(B') (A₂)

in which:
B represents -CH₂CH₂OX',
B' represents -(CH₂)_{z}-Y', with z = 1 or 2,
X' represents the group -CH₂-COOH, CH₂-COOZ', -CH₂CH₂-COOH or -CH₂CH₂-COOZ', or a hydrogen atom,
Y' represents -COOH, -COOZ', or the group CH₂-CHOH-SO₃H or CH₂-CHOH-SO₃Z',
Z' represents an ion derived from an alkali or alkaline-earth metal, such as sodium, an ammonium ion or an ion derived from an organic amine.
Rₐ' represents a C₁₀-C₃₀ alkyl or alkenyl group of an acid Rₐ'-COOH preferably present in hydrolyzed coconut oil or in hydrolyzed linseed oil, an alkyl group, in particular a C₁₇ alkyl group and its iso form, or an unsaturated C₁₇ group.

These compounds of formulae (A1) and (A2) are classified in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid and cocoamphodipropionic acid.

By way of example, mention may be made of the cocoamphodiacetate sold by the company Rhodia under the trade name Miranol® C2M Concentrate.

Rₐ"-NHCH(Y")-(CH₂)ₙCONH(CH₂)ₙ'-N(R_{d})(Rₑ) (A3)

in which formula:
- Y" represents the group -COOH, -COOZ",-CH₂-CH(OH)SO₃H or the group -CH₂CH(OH)SO₃-Z";
- R_{d} and Rₑ represent, independently of each other, a C₁-C₄ alkyl or hydroxyalkyl radical;
- Z" represents a cationic counterion derived from an alkali metal or alkaline-earth metal, such as sodium, an ammonium ion or an ion derived from an organic amine;
- Rₐ" represents a C₁₀-C₃₀ alkyl or alkenyl group of an acid Rₐ"-COOH which is preferably present in coconut oil or in hydrolyzed linseed oil;
- n and n' denote, independently of each other, an integer ranging from 1 to 3.

Among the compounds of formula (A3I), mention may be made of the compound classified in the CTFA dictionary under the name sodium diethylaminopropyl cocoaspartamide and sold by the company Chimex under the name Chimexane HB.

Among the abovementioned amphoteric or zwitterionic surfactants, it is preferred to use (C₈-C₂₀ alkyl)betaines such as cocoylbetaine, (C₈-C₂₀ alkyl)amido(C₃-C₈ alkyl)betaines such as cocoylamidopropylbetaine, and mixtures thereof. More preferentially, the amphoteric or zwitterionic surfactant(s) are chosen from cocoylamidopropylbetaine and cocoylbetaine.

The nonionic surfactant(s) in the compositions of the present invention are especially described, for example, in the Handbook of Surfactants by M.R. Porter, published by Blackie & Son (Glasgow and London), 1991, pp. 116-178. They are especially chosen from fatty alcohols, fatty α-diols, fatty (C₁-C₂₀)alkylphenols and fatty acids, these compounds being ethoxylated, propoxylated or glycerolated and containing at least one fatty chain comprising, for example, from 8 to 18 carbon atoms, the number of ethylene oxide or propylene oxide groups possibly ranging especially from 1 to 200, and the number of glycerol groups possibly ranging especially from 1 to 30.

Mention may also be made of condensates of ethylene oxide and of propylene oxide with fatty alcohols, ethoxylated fatty amides preferably having from 1 to 30 ethylene oxide units, polyglycerolated fatty amides comprising on average from 1 to 5 glycerol groups, and in particular from 1.5 to 4, ethoxylated fatty acid esters of sorbitan containing from 1 to 30 ethylene oxide units, fatty acid esters of sucrose, fatty acid esters of polyethylene glycol, (C₆-C₂₄ alkyl)polyglycosides, oxyethylenated plant oils such as oxyethylenated castor oil, N-(C₆-C₂₄ alkyl)glucamine derivatives, amine oxides such as (C₁₀-C₁₄ alkyl)amine oxides or N-(C₁₀-C₁₄ acyl)aminopropylmorpholine oxides.

The cationic surfactant(s) that can be used in the composition according to the invention are generally chosen from optionally polyoxyalkylenated primary, secondary or tertiary fatty amine salts, quaternary ammonium salts, and mixtures thereof.

Examples of quaternary ammonium salts that may especially be mentioned include:
- those corresponding to the general formula (X) below: 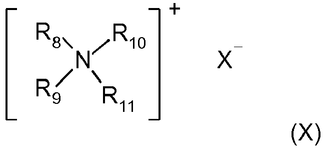 in which the groups R₈ to R₁₁, which may be identical or different, represent a linear or branched aliphatic group comprising from 1 to 30 carbon atoms or an aromatic group, such as aryl or alkylaryl, at least one of the groups R₈ to R₁₁ comprising from 8 to 30 carbon atoms and preferably from 12 to 24 carbon atoms. The aliphatic groups may comprise heteroatoms such as, in particular, oxygen, nitrogen, sulfur and halogens.
   The aliphatic groups are chosen, for example, from C₁-C₃₀ alkyl, C₁-C₃₀ alkoxy, polyoxy(C₂-C₆)alkylene, C₁-C₃₀ alkylamide, (C₁₂-C₂₂)alkylamido(C₂-C₆)alkyl, (C₁₂-C₂₂)alkyl acetate and C₁-C₃₀ hydroxyalkyl groups; X⁻ is an anion chosen from the group of halides, phosphates, acetates, lactates, (C₁-C₄)alkyl sulfates, and (C₁-C₄)alkylsufonates or (C₁-C₄)alkylarylsulfonates.
   Preference is given, among the quaternary ammonium salts of formula (X), on the one hand, to tetraalkylammonium chlorides, such as, for example, dialkyldimethyl-ammonium or alkyltrimethylammonium chlorides in which the alkyl group comprises approximately from 12 to 22 carbon atoms, in particular behenyltrimethylammonium chloride, distearyldimethylammonium chloride, cetyltrimethylammonium chloride or benzyldimethylstearylammonium chloride, or also, on the other hand, to distearoylethylhydroxyethylmethylammonium methosulfate, dipalmitoylethylhydroxyethylammonium methosulfate or distearoylethylhydroxyethylammonium methosulfate, or also, finally, to palmitylamidopropyltrimethylammonium chloride or stearamidopropyldimethyl(myristyl acetate)ammonium chloride, sold under the name Ceraphyl® 70 by the company Van Dyk.
- quaternary ammonium salts of imidazoline, for instance those of formula (XI) below: 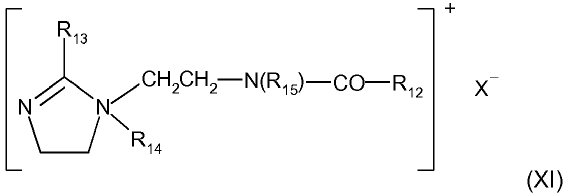 in which
   R₁₂ represents an alkenyl or alkyl group comprising from 8 to 30 carbon atoms, for example derived from tallow fatty acids,
   R₁₃ represents a hydrogen atom, a C₁-C₄ alkyl group or an alkenyl or alkyl group comprising from 8 to 30 carbon atoms,
   R₁₄ represents a C₁-C₄ alkyl group,
   R₁₅ represents a hydrogen atom, a C₁-C₄ alkyl group, X⁻ is an anion chosen from the group of halides, phosphates, acetates, lactates, (C₁-C₄)alkyl sulfates and (C₁-C₄)alkylsulfonates or (C₁-C₄)alkylarylsulfonates.

   Preferably, R₁₂ and R₁₃ denote a mixture of alkenyl or alkyl groups comprising from 12 to 21 carbon atoms, for example derived from tallow fatty acids, R₁₄ denotes a methyl group and R₁₅ denotes a hydrogen atom. Such a product is sold, for example, under the name Rewoquat® W 75 by the company Rewo.
- quaternary diammonium or triammonium salts, particularly of formula (XII) below: 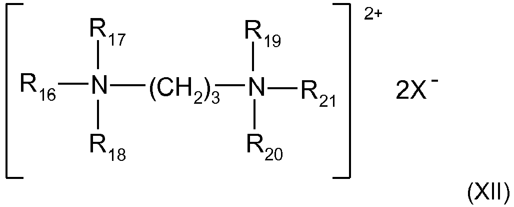
   in which R₁₆ denotes an alkyl group comprising approximately from 16 to 30 carbon atoms, which is optionally hydroxylated and/or interrupted with one or more oxygen atoms,
   R₁₇ is chosen from hydrogen, an alkyl group comprising from 1 to 4 carbon atoms or a group -(CH₂)₃-N⁺(R₁₆ₐ)(R₁₇ₐ)(R₁₈ₐ),
   R₁₆ₐ, R₁₇ₐ, R₁₈ₐ, R₁₈, R₁₉, R₂₀ and R₂₁, which may be identical or different, are chosen from hydrogen or an alkyl group comprising from 1 to 4 carbon atoms, and
   X⁻ is an anion chosen from the group of halides, acetates, phosphates, nitrates, (C₁-C₄)alkyl sulfates, (C₁-C₄)alkyl- or (C₁-C₄)alkylarylsulfonates, in particular methyl sulfate and ethyl sulfate.

   Such compounds are, for example, Finquat CT-P, sold by the company Finetex (Quaternium 89), and Finquat CT, sold by the company Finetex (Quaternium 75).
- quaternary ammonium salts comprising one or more ester functional groups, such as those of formula (XIII) below: 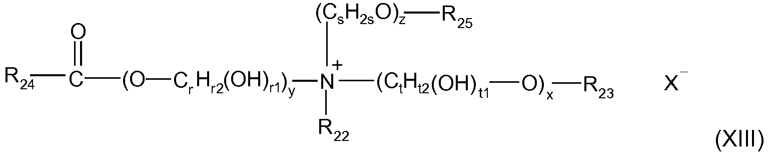 in which:
   R₂₂ is chosen from C₁-C₆ alkyl groups and C₁-C₆ hydroxyalkyl or dihydroxyalkyl groups,
   R₂₃ is chosen from:
- the group 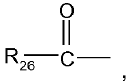
- saturated or unsaturated, linear or branched C₁-C₂₂ hydrocarbon-based groups R₂₇,
- a hydrogen atom,
   R₂₅ is chosen from:
- the group 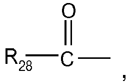
- saturated or unsaturated, linear or branched C₁-C₆ hydrocarbon-based groups R₂₉,
- a hydrogen atom,
   R₂₄, R₂₆ and R₂₈, which may be identical or different, are chosen from saturated or unsaturated, linear or branched C₇-C₂₁ hydrocarbon-based groups;
   r, s and t, which may be identical or different, are integers having values from 2 to 6,
   r1 and t1, which may be identical or different, have the values 0 or 1,
   r2 + r1 = 2 r and t1 + t2 = 2 t,
   y is an integer ranging from 1 to 10,
   x and z, which may be identical or different, are integers having values from 0 to 10,
   X⁻ is a simple or complex, organic or inorganic anion,
   with the proviso that the sum x + y + z is from 1 to 15, that when x is 0 then R₂₃ denotes R₂₇, and that when z is 0 then R₂₅ denotes R₂₉.

The alkyl groups R₂₂ may be linear or branched, and more particularly linear.

Preferably, R₂₂ denotes a methyl, ethyl, hydroxyethyl or dihydroxypropyl group and more particularly a methyl or ethyl group.

Advantageously, the sum x + y + z has a value from 1 to 10.

When R₂₃ is a hydrocarbon-based group R₂₇, it can be long and have from 12 to 22 carbon atoms or be short and have from 1 to 3 carbon atoms.

When R₂₅ is a hydrocarbon-based group R₂₉, it preferably has from 1 to 3 carbon atoms.

Advantageously, R₂₄, R₂₆ and R₂₈, which may be identical or different, are chosen from saturated or unsaturated, linear or branched C₁₁-C₂₁ hydrocarbon-based groups, and more particularly from saturated or unsaturated, linear or branched C₁₁-C₂₁ alkyl and alkenyl groups.

Preferably, x and z, which may be identical or different, are equal to 0 or 1.

Advantageously, y is equal to 1.

Preferably, r, s and t, which may be identical or different, are equal to 2 or 3, and even more particularly are equal to 2.

The anion X⁻ is preferably a halide, preferably chloride, bromide or iodide, a (C₁-C₄)alkyl sulfate, or a (C₁-C₄)alkylsulfonate or (C₁-C₄)alkylarylsulfonate. However, use may be made of methanesulfonate, phosphate, nitrate, tosylate, an anion derived from an organic acid, such as acetate or lactate, or any other anion that is compatible with the ammonium bearing an ester function.

The anion X⁻ is more particularly still chloride, methyl sulfate or ethyl sulfate.

Use is made more particularly, in the composition according to the invention, of the ammonium salts of formula (XIII) in which:
- R₂₂ denotes a methyl or ethyl group,
- x and y are equal to 1,
- z is equal to 0 or 1,
- r, s and t are equal to 2,
- R₂₃ is chosen from:
- the group 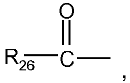
- methyl, ethyl or C₁₄-C₂₂ hydrocarbon-based groups,
- a hydrogen atom,
- R₂₅ is chosen from:
- the group 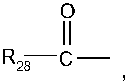
- a hydrogen atom,
- R₂₄, R₂₆ and R₂₈, which may be identical or different, are chosen from linear or branched, saturated or unsaturated C₁₃-C₁₇ hydrocarbon-based groups, and preferably from linear or branched, saturated or unsaturated C₁₃-C₁₇ alkyl and alkenyl groups.

Advantageously, the hydrocarbon-based groups are linear.

Among the compounds of formula (XIII), examples that may be mentioned include salts, especially the chloride or methyl sulfate, of diacyloxyethyldimethylammonium, diacyloxyethylhydroxyethylmethylammonium, monoacyloxyethyldihydroxyethylmethylammonium, triacyloxyethylmethylammonium or monoacyloxyethylhydroxyethyldimethylammonium, and mixtures thereof. The acyl groups preferably contain 14 to 18 carbon atoms and are obtained more particularly from a plant oil, such as palm oil or sunflower oil. When the compound contains several acyl groups, these groups may be identical or different.

These products are obtained, for example, by direct esterification of triethanolamine, triisopropanolamine, an alkyldiethanolamine or an alkyldiisopropanolamine, which are optionally oxyalkylenated, with fatty acids or with fatty acid mixtures of plant or animal origin, or by transesterification of the methyl esters thereof. This esterification is followed by a quaternization by means of an alkylating agent such as an alkyl halide, preferably methyl or ethyl halide, a dialkyl sulfate, preferably dimethyl or diethyl sulfate, methyl methanesulfonate, methyl paratoluenesulfonate, glycol chlorohydrin or glycerol chlorohydrin.

Such compounds are sold, for example, under the names Dehyquart® by the company Henkel, Stepanquat® by the company Stepan, Noxamium® by the company Ceca or Rewoquat® WE 18 by the company Rewo-Witco.

The composition according to the invention may contain, for example, a mixture of quaternary ammonium monoester, diester and triester salts with a weight majority of diester salts.

It is also possible to use the ammonium salts containing at least one ester function that are described in patents US-A-4 874 554 and US-A-4 137 180.

Use may also be made of behenoylhydroxypropyltrimethylammonium chloride, for example, sold by the company KAO under the name Quartamin BTC 131.

Preferably, the ammonium salts containing at least one ester function contain two ester functions.

Among the cationic surfactants, it is more particularly preferred to choose cetyltrimethylammonium, behenyltrimethylammonium and dipalmitoylethylhydroxyethylmethylammonium salts, and mixtures thereof, and more particularly behenyltrimethylammonium chloride, cetyltrimethylammonium chloride, and dipalmitoylethylhydroxyethylammonium methosulfate, and mixtures thereof.

Preferably, when they are present, the surfactant(s) are chosen from nonionic surfactants and cationic surfactants.

When the composition comprises one or more surfactants, their content may preferably range from 0.05% to 20% by weight, more preferentially from 0.1% to 10% by weight and better still from 0.5% to 5% by weight relative to the total weight of the composition.

The composition according to the invention may also comprise one or more additives chosen from fatty subtances, silicones, plasticizers, co-thickeners other than those described previously, sunscreens, antioxidants, acids, bases, fragrances, preserving agents, mineral fillers, nacreous agents, opacifiers, dyes and glitter flakes.

A person skilled in the art will take care to select the optional additives and the amounts thereof so that they do not interfere with the properties of the compositions of the present invention.

These additives may each be present in the composition according to the invention in a content ranging from 0 to 20% by weight, relative to the total weight of the composition.

Particularly preferably, the composition according to the invention also comprises water, which advantageously represents from 30% to 99% by weight, preferably from 35% to 95% by weight and better still from 40% to 85% by weight, relative to the total weight of the composition.

The composition may also comprise one or more water-soluble organic solvents (solubility of greater than or equal to 5% by weight in water at 25°C and at atmospheric pressure).

Examples of water-soluble organic solvents that may be mentioned include linear or branched and preferably saturated monoalcohols or diols, comprising 2 to 10 carbon atoms, such as ethyl alcohol, isopropyl alcohol, hexylene glycol (2-methyl-2,4-pentanediol), neopentyl glycol and 3-methyl-1,5-pentanediol, butylene glycol, dipropylene glycol and propylene glycol; aromatic alcohols such as phenylethyl alcohol; polyols containing more than two hydroxyl functions, such as glycerol; polyol ethers, for instance ethylene glycol monomethyl, monoethyl or monobutyl ether, propylene glycol or ethers thereof, for instance propylene glycol monomethyl ether; and also diethylene glycol alkyl ethers, especially C₁ to C₄ alkyl ethers, for instance diethylene glycol monoethyl ether or monobutyl ether, alone or as a mixture.

The water-soluble organic solvents, when they are present, generally represent from 1% to 40% by weight and preferably from 5% to 35% by weight relative to the total weight of the composition.

Preferably, in the composition according to the invention, the pH ranges from 3 to 11 and preferably from 4 to 9.

The composition according to the invention may be in the form of liquids that are more or less thickened, gels, creams, pastes or mousses.

Preferably, the composition according to the invention is in gel form.

In a preferred variant, the viscosity of the compositions of the invention is greater than or equal to 0.1 Pa.s, better still greater than or equal to 0.2 Pa.s and even better still greater than or equal to 0.5 Pa.s at 25°C and at a shear rate of 1 s⁻¹. This viscosity can be determined with a rheometer having cone-plate geometry.

Another subject of the invention is the use of the composition as defined previously for styling the hair.

A final subject of the invention is a method for treating keratin fibres, preferably the hair, comprising the application of the composition as defined previously to the keratin fibres, with or without final rinsing after an optional leave-on time.

Preferably, the application of the composition of the invention is not rinsed off.

The composition according to the invention may be applied at room temperature (T = 25°C) or with a supply of heat (45 to 230°C).

The examples that follow are given purely as illustrations of the present invention.

### EXAMPLE

The following compositions A to C according to the invention were prepared from the ingredients indicated in the table below. The concentrations are expressed as weight percentages of active material in the final composition.

| **Ingredients** | **A** | **B** | **C** |
|---|---|---|---|
| Acrylates Copolymer⁽¹⁾ | 1.5 | 1.5 | 1.5 |
| Acrylates/hydroxyacrylates copolymer⁽²⁾ | 3.8 | 3.8 | 3.8 |
| Acrylates/Steareth-20 methacrylate crosslinked copolymer ⁽³⁾ | 2.0 | 2.0 | 2.0 |
| Sorbitol as an aqueous 70% solution | 1.4 | 1.4 | - |
| Glycerol | - | - | 2.0 |
| Oxyethylenated (40 EO) hydrogenated castor oil | 1.0 | 1.0 | 1.0 |
| Propylene glycol | 3.0 | 3.0 | 3.0 |
| Ethanol | - | 2.0 | - |
| 1,2-Octanediol (Caprylyl glycol) | 1.0 | 1.0 | 1.0 |
| Triethanolamine | 2.5 | 2.5 | 2.5 |
| Preserving agents | 0.5 | 0.5 | 0.5 |
| Fragrance | 0.3 | 0.3 | 0.3 |
| 2-Amino-2-methyl-1-propanol | qs | qs | qs |
| Deionized water | qs for 100 g | qs for 100 g | qs for 100 g |

| | | | |
|---|---|---|---|
| ⁽¹⁾ Aquastyle SH 100 sold by the company Ashland ⁽²⁾ Acudyne 180 sold by the company Rohm & Haas ⁽³⁾ Aculyn 88 sold by the company Rohm & Haas | | | |

The compositions A to C according to the invention are in the form of a gel and are used as styling gel at room temperature (25°C). They are applied to dry or towel-dried clean hair in an amount of 5 g of composition per head.

The compositions A to C according to the invention have a texture that results in improved usage qualities that enable good use of the gel, in particular with easier uptake and distribution. This enables good spreading of the gel over the hair.

Furthermore, these compositions make it possible to obtain high styling performance, i.e. good fixing and a long-lasting hold of the hairstyle.

The composition D according to the invention and the comparative composition E that follow were also prepared, from the ingredients indicated in the table below:
The concentrations are expressed as weight percentages of active material in the final composition.

| **Ingredients** | **D** | **E** |
|---|---|---|
| Acrylates/hydroxyacrylates copolymer⁽²⁾ | 3.8 | 3.8 |
| Acrylates Copolymer⁽¹⁾ | 1.5 | - |
| Acrylates/Steareth-20 methacrylate crosslinked copolymer ⁽³⁾ | 2.0 | 3.5 |
| Triethanolamine | 2.5 | 2.5 |
| 2-Amino-2-methyl-1-propanol | 0.5 | 0.5 |
| Deionized water | qs for 100 g | qs for 100 g |
| pH | 7.3 | 7.4 |

| | | |
|---|---|---|
| ⁽¹⁾ Aquastyle SH 100 sold by the company Ashland ⁽²⁾ Acudyne 180 sold by the company Rohm & Haas ⁽³⁾ Aculyn 88 sold by the company Rohm & Haas | | |

With the composition D according to the invention, a gel is obtained which has a texture that enables good grasping and an easy application of the product to the hair. With the comparative composition E, an excessively thick gel is obtained which is difficult to apply to the hair.

The two compositions were applied to a malleable head, the composition D having been applied to one half of the head and the composition E having been applied to the other half.

After 12 passes of a hand on each side, a persistence of the film formed by the composition D of the invention is observed whereas the film formed by the composition E disintegrated. The hair treated with the composition D is still coated whereas the hair treated with the composition E is individualized.

A better form retention of the hairstyle is obtained with the composition D than with the composition E.

The composition D of the invention therefore makes it possible to obtain a styling gel that has an improved resistance to mechanical friction.

## Claims

1. Composition comprising:
(i) one or more anionic associative polymers (i) comprising at least one unit of formula (I) and at least one unit of formula (II) below: 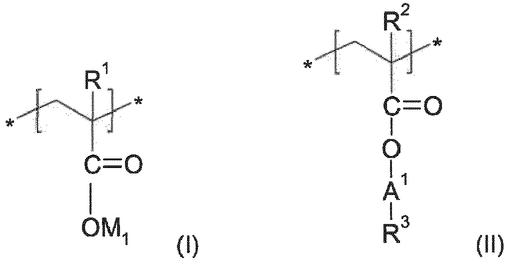
with R¹ and R², independently of each other, representing a hydrogen atom or a methyl group,
R³ representing a C₈-C₃₀ alkyl radical,
M₁ representing a hydrogen atom or a physiologically acceptable cation,
A¹ representing a group -(CH₂CH₂O)ₓ-, x being an integer ranging from 5 to 35, a group -(CH₂CHMeO)_{y}-, y being an integer ranging from 5 to 35, or a group -(CH₂CH₂O)ₓ-(CH₂CHMeO)_{y}-, the sum x + y being an integer ranging from 5 to 35 with x and y being greater than 0,
(ii) one or more anionic fixing polymers comprising at least one unit of formula (IV) and at least one unit of formula (V) below: 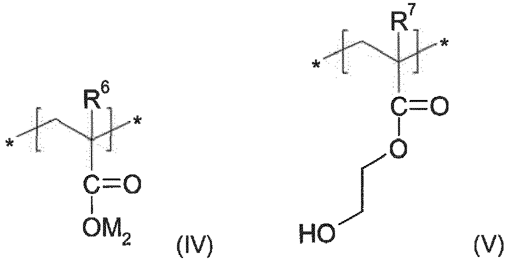
with R⁶ and R⁷, independently of each other, representing a hydrogen atom or a methyl group, and
M₂ representing a hydrogen atom or a physiologically acceptable cation;
(iii) one or more anionic polymers, different from the anionic associative polymers (i) and from the anionic fixing polymers (ii), comprising:
- at least one (meth)acrylic acid monomer,
- at least one C₁-C₄ alkyl (meth)acrylate monomer,
- at least one (meth)acrylic ester monomer with a hydrophobic chain.

2. Composition according to Claim 1, **characterized in that** the anionic associative polymer(s) (i) are crosslinked.

3. Composition according to Claim 1 or 2, **characterized in that** the anionic associative polymer(s) (i) also comprise at least one unit of formula (III) below: 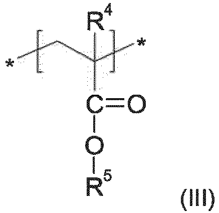
with R⁴ representing a hydrogen atom or a methyl group, and
R⁵ representing a C₁ to C₄ alkyl group, and in particular a methyl or ethyl group.

4. Composition according to any one of the preceding claims, **characterized in that** the anionic associative polymer(s) (i) represent from 0.1 % to 20% by weight, preferably from 0.5% to 15% by weight, and in particular from 1% to 10% by weight and better still from 1% to 5% by weight, relative to the total weight of the composition.

5. Composition according to any one of the preceding claims, **characterized in that** the anionic fixing polymer(s) (ii) also comprise at least one unit of formula (III) below: 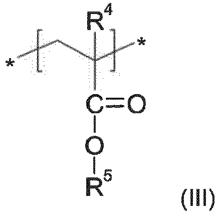
with R⁴ representing a hydrogen atom or a methyl group, and
R⁵ representing a C₁ to C₄ alkyl group, in particular a methyl, ethyl or n-butyl group.

6. Composition according to any one of the preceding claims, **characterized in that** the anionic fixing polymer(s) (ii) are non-crosslinked.

7. Composition according to any one of the preceding claims, **characterized in that** the anionic fixing polymer(s) (ii) represent from 0.1% to 20% by weight, preferably from 0.2% to 15% by weight, more preferentially from 0.5% to 10% and in particular from 1% to 8% by weight, relative to the total weight of the composition.

8. Composition according to any one of the preceding claims, **characterized in that** the anionic polymer (iii) different from the anionic associative polymers (i) and from the anionic fixing polymers (ii) is crosslinked.

9. Composition according to any one of the preceding claims, **characterized in that** the anionic polymer (iii) comprises less than 10% by weight of (meth)acrylic ester monomer with a hydrophobic chain, preferably between 1% and 5% by weight relative to the total weight of the polymer.

10. Composition according to any one of the preceding claims, **characterized in that** the anionic polymer (iii) comprises from 45% to 60% by weight of C₁-C₄ alkyl (meth)acrylate monomer and from 30% to 40% by weight of (meth)acrylic acid monomer relative to the total weight of the polymer.

11. Composition according to any one of the preceding claims, **characterized in that** the anionic polymer (iii) comprises at least one (meth)acrylic ester monomer comprising an oxyalkylenated hydrophobic chain.

12. Composition according to any one of the preceding claims, **characterized in that** the anionic polymer (iii) comprises at least one (meth)acrylic ester monomer of formula (VI): 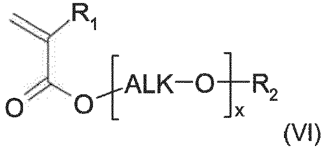 in which
- denotes a hydrogen atom or a linear or branched C₁-C₆ alkyl radical, preferably methyl;
- R₂ denotes a saturated or unsaturated, linear or branched, hydrophobic hydrocarbon-based radical comprising from 8 to 50 carbon atoms and more preferentially from 10 to 30 carbon atoms and more preferentially still from 12 to 22 carbon atoms;
- ALK is a saturated or unsaturated, linear or branched hydrocarbon-based divalent group comprising from 1 to 10 carbon atoms, preferably from 1 to 6 carbon atoms, preferably 2 or 3 carbon atoms such as the ethylene group,
- x denotes an integer ranging from 0 to 100, preferably from 1 to 50, better still from 1 to 20.

13. Composition according to any one of the preceding claims, **characterized in that** the anionic polymer(s) (iii) represent from 0.1% to 20% by weight, preferably from 0.5% to 15% by weight, more preferentially from 1% to 10%, and in particular from 1% to 5% by weight, relative to the total weight of the composition.

14. Use of the composition as defined in any one of the preceding claims, for styling keratin fibres, preferably the hair.

15. Method for treating keratin fibres, preferably the hair, comprising the application of the composition as defined in any one of Claims 1 to 13 to the keratin fibres.

## Patentansprüche

1. Zusammensetzung, umfassend:
(i) ein oder mehrere anionische assoziative Polymere (i), die mindestens eine Einheit der Formel (I) und mindestens eine Einheit der Formel (II) unten umfassen: 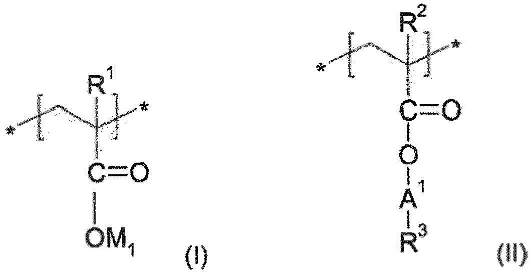
wobei und R² unabhängig voneinander für ein Wasserstoffatom oder eine Methylgruppe stehen,
R³ für einen C₈-C₃₀-Alkylrest steht,
M₁ für ein Wasserstoffatom oder ein physiologisch unbedenkliches Kation steht,
A¹ für eine Gruppe - (CH₂CH₂O)ₓ-, wobei x für eine ganze Zahl im Bereich von 5 bis 35 steht, eine Gruppe -(CH₂CHMeO)_{y}-, wobei y für eine ganze Zahl im Bereich von 5 bis 35 steht, oder eine Gruppe -(CH₂CH₂O)ₓ-(CH₂CHMeO)_{y}-, wobei die Summe x + y eine ganze Zahl im Bereich von 5 bis 35 ist, wobei x und y größer als 0 sind, steht,
(ii) ein oder mehrere anionische fixierende Polymere, die mindestens eine Einheit der Formel (IV) und mindestens eine Einheit der Formel (V) unten umfassen: 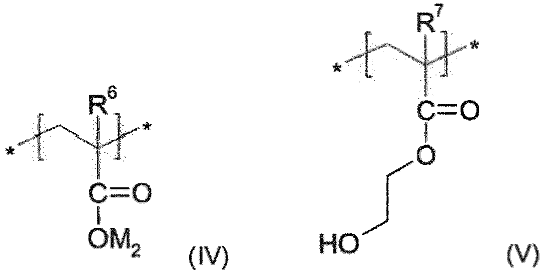
wobei R⁶ und R⁷ unabhängig voneinander für ein Wasserstoffatom oder eine Methylgruppe stehen und
M₂ für ein Wasserstoffatom oder ein physiologisch unbedenkliches Kation steht;
(iii) ein oder mehrere anionische Polymere, die von den anionischen assoziativen Polymeren (i) und von den anionischen fixierenden Polymeren (ii) verschieden sind, umfassend:
- mindestens ein (Meth)acrylsäure-Monomer,
- mindestens ein C₁-C₄-Alkyl(meth)acrylat-Monomer,
- mindestens ein (Meth)acrylsäureester-Monomer mit einer hydrophoben Kette.

2. Zusammensetzung nach Anspruch 1, **dadurch gekennzeichnet, dass** das anionische assoziative Polymer bzw. die anionischen assoziativen Polymere (i) vernetzt ist bzw. sind.

3. Zusammensetzung nach Anspruch 1 oder 2, **dadurch gekennzeichnet, dass** das anionische assoziative Polymer bzw. die anionischen assoziativen Polymere (i) außerdem mindestens eine Einheit der Formel (III) unten umfasst bzw. umfassen: 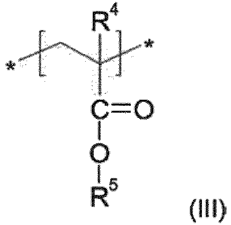
wobei R⁴ für ein Wasserstoffatom oder eine Methylgruppe steht und
R⁵ für eine C₁- bis C₄-Alkylgruppe und insbesondere eine Methyl- oder Ethylgruppe steht.

4. Zusammensetzung nach einem der vorhergehenden Ansprüche, **dadurch gekennzeichnet, dass** das anionische assoziative Polymer bzw. die anionischen assoziativen Polymere (i) 0,1 bis 20 Gew.-%, vorzugsweise 0,5 bis 15 Gew.-% und insbesondere 1 bis 10 Gew.-% und noch besser 1 bis 5 Gew.-%, bezogen auf das Gesamtgewicht der Zusammensetzung, ausmacht bzw. ausmachen.

5. Zusammensetzung nach einem der vorhergehenden Ansprüche, **dadurch gekennzeichnet, dass** das anionische fixierende Polymer bzw. die anionischen fixierenden Polymere (ii) außerdem mindestens eine Einheit der Formel (III) unten umfasst bzw. umfassen: 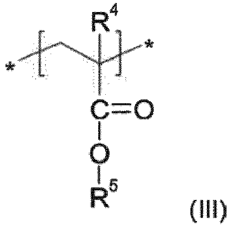
wobei R⁴ für ein Wasserstoffatom oder eine Methylgruppe steht und
R⁵ für eine C₁- bis C₄-Alkylgruppe, insbesondere eine Methyl-, Ethyl- oder n-Butylgruppe, steht.

6. Zusammensetzung nach einem der vorhergehenden Ansprüche, **dadurch gekennzeichnet, dass** das anionische fixierende Polymer bzw. die anionischen fixierenden Polymere (ii) unvernetzt ist bzw. sind.

7. Zusammensetzung nach einem der vorhergehenden Ansprüche, **dadurch gekennzeichnet, dass** das anionische fixierende Polymer bzw. die anionischen fixierenden Polymere (ii) 0,1 bis 20 Gew.-%, vorzugsweise 0,2 bis 15 Gew.-%, weiter bevorzugt 0,5 bis 10 Gew.-% und insbesondere 1 bis 8 Gew.-%, bezogen auf das Gesamtgewicht der Zusammensetzung, ausmacht bzw. ausmachen.

8. Zusammensetzung nach einem der vorhergehenden Ansprüche, **dadurch gekennzeichnet, dass** das anionische Polymer (iii), das von den anionischen assoziativen Polymeren (i) und von den anionischen fixierenden Polymeren (ii) verschieden ist, vernetzt ist.

9. Zusammensetzung nach einem der vorhergehenden Ansprüche, **dadurch gekennzeichnet, dass** das anionische Polymer (iii) weniger als 10 Gew.-% (Meth)acrylsäureester-Monomer mit einer hydrophoben Kette, vorzugsweise zwischen 1 und 5 Gew.-%, bezogen auf das Gesamtgewicht des Polymers, umfasst.

10. Zusammensetzung nach einem der vorhergehenden Ansprüche, **dadurch gekennzeichnet, dass** das anionische Polymer (iii) 45 bis 60 Gew.-% C₁-C₄-Alkyl(meth)acrylat-Monomer und 30 bis 40 Gew.-% (Meth)acrylsäure-Monomer, bezogen auf das Gesamtgewicht des Polymers, umfasst.

11. Zusammensetzung nach einem der vorhergehenden Ansprüche, **dadurch gekennzeichnet, dass** das anionische Polymer (iii) mindestens ein (Meth)-acrylsäureester-Monomer mit einer oxyalkylenierten hydrophoben Kette umfasst.

12. Zusammensetzung nach einem der vorhergehenden Ansprüche, **dadurch gekennzeichnet, dass** das anionische Polymer (iii) mindestens ein (Meth)-acrylsäure-Monomer der Formel (VI) umfasst: 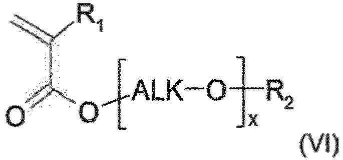 in der
- R₁ für ein Wasserstoffatom oder einen linearen oder verzweigten C₁-C₆-Alkylrest, vorzugsweise Methyl, steht;
- R₂ für einen gesättigten oder ungesättigten, linearen oder verzweigten, hydrophoben Rest auf Kohlenwasserstoffbasis mit 8 bis 50 Kohlenstoffatomen und weiter bevorzugt 10 bis 30 Kohlenstoffatomen und noch weiter bevorzugt 12 bis 22 Kohlenstoffatomen steht;
- ALK für eine gesättigte oder ungesättigte, lineare oder verzweigte zweiwertige Gruppe auf Kohlenwasserstoffbasis mit 1 bis 10 Kohlenstoffatomen, vorzugsweise 1 bis 6 Kohlenstoffatomen, vorzugsweise 2 oder 3 Kohlenstoffatomen, wie die Ethylengruppe, steht,
- x für eine ganze Zahl im Bereich von 0 bis 100, vorzugsweise von 1 bis 50, noch besser von 1 bis 20, steht.

13. Zusammensetzung nach einem der vorhergehenden Ansprüche, **dadurch gekennzeichnet, dass** das anionische Polymer bzw. die anionischen Polymere (iii) 0,1 bis 20 Gew.-%, vorzugsweise 0,5 bis 15 Gew.-%, weiter bevorzugt 1 bis 10 Gew.-% und insbesondere 1 bis 5 Gew.-%, bezogen auf das Gesamtgewicht der Zusammensetzung, ausmacht bzw. ausmachen.

14. Verwendung der Zusammensetzung gemäß einem der vorhergehenden Ansprüche zum Frisieren von Keratinfasern, vorzugsweise dem Haar.

15. Verfahren zum Behandeln von Keratinfasern, vorzugsweise dem Haar, bei dem man die Zusammensetzung gemäß einem der Ansprüche 1 bis 13 auf die Keratinfasern aufbringt.

## Revendications

1. Composition comprenant :
(i) un ou plusieurs polymères associatifs anioniques (i) comprenant au moins un motif de formule (I) et au moins un motif de formule (II) suivantes : 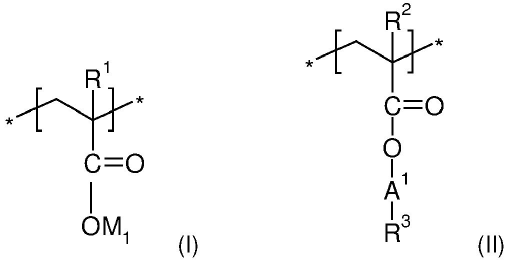
avec R¹ et R² représentant, indépendamment l'un de l'autre, un atome d'hydrogène ou un groupe méthyle,
R³ représentant un radical alkyle en C₈ à C₃₀,
M₁ représentant un atome d'hydrogène ou un cation physiologiquement acceptable,
A¹ représentant un groupe -(CH₂CH₂O)ₓ-, x étant un nombre entier variant de 5 à 35, un groupe -(CH₂CHMeO)_{y}-, y étant un nombre entier variant de 5 à 35, ou un groupe -(CH₂CH₂O)ₓ-(CH₂CHMeO)_{y}-, la somme x+y étant un nombre entier variant de 5 à 35 avec x et y supérieurs à 0,
(ii) un ou plusieurs polymères fixants anioniques comprenant au moins un motif de formule (IV) et au moins un motif de formule (V) suivantes : 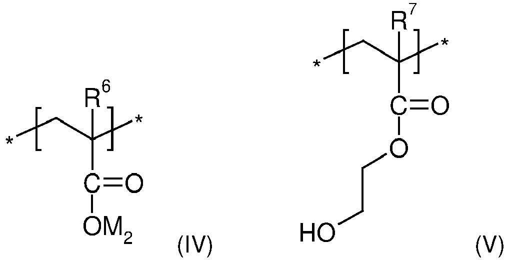
avec R⁶ et R⁷ représentant, indépendamment l'un de l'autre, un atome d'hydrogène ou un groupe méthyle, et
M₂ représentant un atome d'hydrogène ou un cation physiologiquement acceptable ;
(iii) un ou plusieurs polymère(s) anionique(s), différent(s) des polymères associatifs anioniques (i) et des polymères fixants anioniques (ii), comprenant :
- au moins un monomère acide(méth)acrylique,
- au moins un monomère (méth)acrylate d'alkyle en C₁-C₄,
- au moins un monomère ester (méth)acrylique à chaine hydrophobe.

2. Composition selon la revendication 1, **caractérisée en ce que** le ou les polymères associatifs anioniques (i) sont réticulés.

3. Composition selon la revendication 1 ou 2, **caractérisée en ce que** le ou les polymères associatifs anioniques (i) comprennent en plus au moins un motif de formule (III) suivante : 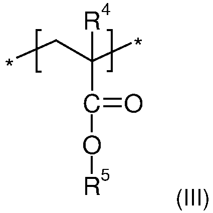
avec R⁴ représentant un atome d'hydrogène ou un groupe méthyle, et
R⁵ représentant un groupe alkyle en C₁ à C₄, et en particulier un groupe méthyle ou éthyle.

4. Composition selon l'une quelconque des revendications précédentes, **caractérisée en ce que** le ou les polymères associatifs anioniques (i) représentent de 0,1 à 20 % en poids, de préférence de 0,5 à 15 % en poids, et en particulier de 1 à 10 % en poids, et mieux de 1 à 5 % en poids par rapport au poids total de la composition.

5. Composition selon l'une quelconque des revendications précédentes, **caractérisée en ce que** le ou les polymères fixants anioniques (ii) comprennent en outre au moins un motif de formule (III) suivante : 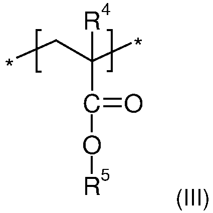
avec R⁴ représentant un atome d'hydrogène ou un groupe méthyle, et
R⁵ représentant un groupe alkyle en C₁ à C₄, en particulier un groupe méthyle, éthyle ou n-butyle.

6. Composition selon l'une quelconque des revendications précédentes, **caractérisée en ce que** le ou les polymères fixants anioniques (ii) sont non-réticulés.

7. Composition selon l'une quelconque des revendications précédentes, **caractérisée en ce que** le ou les polymères fixants anioniques (ii) représentent de 0,1 à 20 % en poids, de préférence de 0,2 à 15 % en poids, plus préférentiellement de 0,5 à 10 %, et en particulier de 1 à 8 % en poids, par rapport au poids total de la composition.

8. Composition selon l'une quelconque des revendications précédentes, **caractérisée en ce que** le polymère anionique (iii) différent des polymères associatifs anioniques (i) et des polymères fixants anioniques (ii), est réticulé.

9. Composition selon l'une quelconque des revendications précédentes, **caractérisée en ce que** le polymère anionique (iii) comprend moins de 10% en poids de monomère ester (méth)acrylique à chaine hydrophobe, de préférence entre 1 et 5% en poids par rapport au poids total du polymère.

10. Composition selon l'une quelconque des revendications précédentes **caractérisée en ce que** le polymère anionique (iii) comprend de 45 à 60% en poids, de monomère (méth)acrylate d'alkyle en C₁-C₄ ; et de 30 à 40% en poids de monomère acide(méth)acrylique par rapport au poids total du polymère.

11. Composition selon l'un quelconque des revendications précédentes, **caractérisée en ce que** le polymère anionique (iii) comprend au moins un monomère ester (méth)acrylique comprenant une chaine hydrophobe oxyalkylénée.

12. Composition selon l'une quelconque des revendications précédentes **caractérisée en ce que** le polymère anionique (iii) comprend au moins un monomère ester (méth)acrylique de formule (VI) : 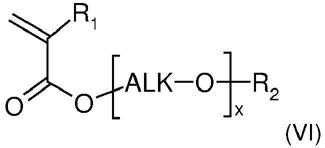 dans laquelle
- désigne un atome d'hydrogène ou un radical alkyle, linéaire ou ramifié en C₁-C₆, de préférence méthyle ;
- R² désigne un radical hydrocarboné hydrophobe, saturé ou insaturé, linéaire ou ramifié, comprenant de 8 à 50 atomes de carbone et plus préférentiellement de 10 à 30 atomes de carbone et encore plus préférentiellement de 12 à 22 atomes de carbone ;
- ALK est un groupe divalent hydrocarboné, saturé ou insaturé, linéaire ou ramifié, comprenant de 1 à 10 atomes de carbone, de préférence de 1 à 6 atomes de carbone, de préférence, 2 ou 3 atomes de carbone tel que le groupe éthylène,
- x désigne un nombre entier allant de 0 à 100, de préférence de 1 à 50, mieux de 1 à 20.

13. Composition selon l'une quelconque des revendications précédentes, **caractérisée en ce que** le ou les polymère(s) anionique(s) (iii) représente(nt) de 0,1 à 20 % en poids, de préférence de 0,5 à 15 % en poids, plus préférentiellement de 1 à 10 %, et en particulier de 1 à 5 % en poids, par rapport au poids total de la composition.

14. Utilisation de la composition telle que définie dans l'une quelconque des revendications précédentes pour le coiffage des fibres kératiniques, de préférence des cheveux.

15. Procédé de traitement des fibres kératiniques, de préférence des cheveux, comprenant l'application sur les fibres kératiniques de la composition telle que définie dans l'une quelconque des revendications 1 à 13.
